(19) Europäisches Patentamt
European Patent Office
Office européen des brevets

(11) Veröffentlichungsnummer: **0 020 941**
**B1**

(12) # EUROPÄISCHE PATENTSCHRIFT

(45) Veröffentlichungstag der Patentschrift: **29.06.83**

(21) Anmeldenummer: **80102320.1**

(22) Anmeldetag: **30.04.80**

(51) Int. Cl.³: **C 07 D 207/06,** C 07 D 207/20, C 07 D 401/12, C 07 D 409/10, A 61 K 31/40 // (C07D401/12, 207/06, 213/65), (C07D409/10, 207/06, 333/24)

(54) **Substituierte 3-(1-Pyrrolidinyl)-5-sulfamoyl-benzoesäure-Derivate, Verfahren zu ihrer Herstellung, pharmazeutische Zubereitungen, die sie enthalten und Verfahren zu deren Herstellung.**

(30) Priorität: **04.05.79 DE 2917997**

(43) Veröffentlichungstag der Anmeldung: **07.01.81 Patentblatt 81/1**

(45) Bekanntmachung des Hinweises auf die Patenterteilung: **29.06.83 Patentblatt 83/26**

(84) Benannte Vertragsstaaten: **AT BE CH DE FR GB IT LI NL SE**

(56) Entgegenhaltungen:
**DE-A-2 419 170**
**DE-A-2 461 601**
**DE-A-2 658 766**
**DE-A-2 718 494**
**DE-A-2 756 783**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt/Main 80 (DE)**

(72) Erfinder: **Merkel, Wulf, Dr., Hattersheimer Strasse 14, D-6233 Kelkheim (Taunus) (DE)**
Erfinder: **Bormann, Dieter, Dr., Am Schieferberg 37, D-6233 Kelkheim (Taunus) (DE)**
Erfinder: **Muschaweck, Roman, Heimchenweg 39, D-6230 Frankfurt am Main 80 (DE)**

**EP 0 020 941 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Substituierte 3-(1-Pyrrolidinyl)-5-sulfamoyl-benzoesäure-Derivate, Verfahren zu ihrer Herstellung pharmazeutische Zubereitungen, die sie enthalten und Verfahren zu deren Herstellung (30.09.82)

Gegenstand der Erfindung sind substituierte Pyrrolidinylsulfamoylbenzoesäurederivate der allgemeinen Formel I

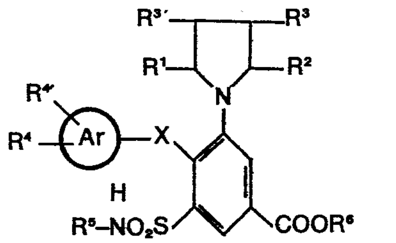

I

worin $R^1$ und $R^2$ gleich oder verschieden sind und Alkyl mit 1–4 C-Atomen bedeuten, wobei einer der Reste $R^1$ oder $R^2$ auch Wasserstoff sein kann,

und $R^3$ und $R^{3'}$ gleich oder verschieden sind und Wasserstoff oder Alkyl mit 1–4 C-Atomen, oder zusammen eine C-C-Bindung bedeuten,

$R^4$ und $R^{4'}$ gleich oder verschieden sind und Wasserstoff, Halogen, $CF_3$, Alkyl oder Alkoxy mit 1–2 C-Atomen, Hydroxy, Amino oder Dimethylamino, in den verschiedenen Positionen des Arylkerns, oder zusammen die 3,4-Methylendioxygruppe bedeuten,

$R^5$ und $R^6$ gleich oder verschieden sind und Wasserstoff, Alkyl mit 1–4 C-Atomen oder Benzyl bedeuten,

Ar Phenyl oder einen 5- oder 6gliedrigen hetero-aromatischen Ring mit einem O-, S- oder N-Atom bedeutet und X entfällt oder Sauerstoff, Schwefel, NH oder $CH_2$ bedeutet, sowie deren pharmazeutisch verträgliche Salze mit Basen oder Säuren.

Wenn X «entfällt», bedeutet dies, dass der Substituent Ar direkt an den Benzoesäurerest gebunden ist und damit ein Biarylsystem bildet.

Die Substituenten $R^1$, $R^2$, $R^3$ und $R^{3'}$ am Pyrrolidinring können in allen möglichen stereoisomeren Stellungen angeordnet sein.

Gegenstand der Erfindung ist weiterhin ein Verfahren zur Herstellung der Verbindungen der Formel I, das dadurch gekennzeichnet ist, dass man 3-Aminobenzoesäurederivate der allgemeinen Formel II

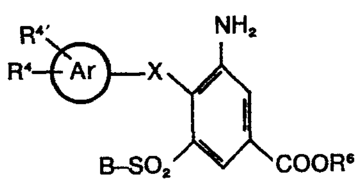

II

in der $R^4$, $R^{4'}$, $R^5$, $R^6$, X und Ar die angegebenen Bedeutungen haben, und B entweder die Gruppe $NHR^5$ oder die Gruppierung III

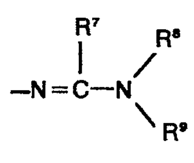

III

bedeutet, wobei die Reste $R^7$, $R^8$, $R^9$ für eine niedere Alkylgruppe stehen, $R^7$ aber auch Wasserstoff sein kann und/oder jeweils zwei der Substituenten auch cyclisch miteinander verbunden sein können, mit 2,5-Dialkoxytetrahydrofuranen der Formel IV

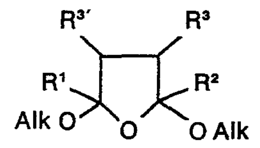

IV

oder mit 1,4-Diketonen der Formel V

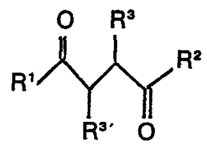

V

in der $R^1$, $R^2$, $R^3$ und $R^{3'}$ die angegebenen Bedeutungen haben und Alk für niederes Alkyl mit 1–4 C-Atomen steht, umsetzt und die erhaltenen Verbindungen der Formel VI

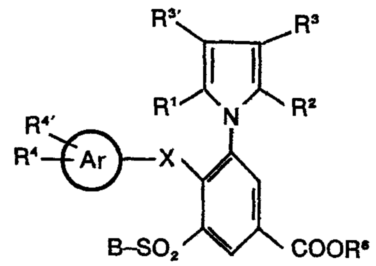

VI

in der die Reste $R^1$–$R^6$ und B die oben genannte Bedeutung haben, reduziert und vor oder nach der Reduktion gegebenenfalls hydrolysiert und erhaltene Verbindungen der Formel VI mit $R^6$ = H gegebenenfalls verestert und/oder die erhaltenen Verbindungen I in pharmazeutisch verträgliche Salze mit Säuren oder Basen überführt.

Die als Ausgangsstoffe verwendeten Aminoverbindungen der Formel II sind literaturbekannt oder können analog den in der Literatur angegebenen Wegen hergestellt werden. Gleichfalls literaturbekannt sind die Tetrahydrofurane IV sowie die Diketone V.

Bedeutet B in den Formeln II und VI die NHR-Gruppe und $R^6$ Wasserstoff, so gelangt man durch Reduktion der Verbindungen der Formel VI direkt zu Verbindungen der Formel I mit $R^6$ = H.

Falls $R^6$ nicht Wasserstoff und/oder B die Gruppierung III bedeuten, werden die Verbindungen der Formel VI entweder anschliessend hydrolysiert und die so erhaltenen Säuren der allgemeinen Formel VI mit $R^6$ = H katalytisch zu Verbindungen der allgemeinen Formel I mit $R^6$ = H reduziert oder es wird zunächst katalytisch hydriert und anschliessend hydrolysiert, da die geschützte Sulfamoylgruppe bei der katalytischen Hydrierung nicht angegriffen wird.

Als pharmazeutisch unbedenkliche Salze kommen vor allem Alkali- und Erdalkalisalze in Betracht, wie beispielsweise Na-, K-, $NH_4$- oder Ca-Salze, aber auch Salze organischer Basen, wie beispielsweise das Äthanolamin-Salz.

Als pharmazeutisch unbedenkliche Ester seien die niedrigen Alkyl- beispielsweise die Methyl-, Ethyl- oder Isopropylester genannt.

Die Umsetzung der Amine II mit den Tetrahydrofuranen IV erfolgt in an sich bekannter Weise gemäss Acta Chem. Scand. 6, 867 (1952). Sie wird mit Hilfe von verdünnter Mineralsäure oder organischen Säuren, vorzugsweise Eisessig durchgeführt. Als besonders vorteilhaft hat sich die Verwendung eines Gemisches eines chlorierten Lösungsmittels, vorzugsweise Chloroform oder Methylenchlorid, mit Eisessig erwiesen. Die Umsetzungen können in der Siedehitze des jeweiligen Lösungsmittels oder Lösungsmittelgemisches oder auch bei Raumtemperatur durchgeführt werden.

Die Umsetzung der Amine II mit den Diketonen V erfolgt ebenfalls in an sich bekannter Weise gemäss Chem. Ber. 109, 3426 (1976), sie wird in schwach organischen Säuren, vorzugsweise Eisessig, oder in einem neutralen organischen Lösungsmittel, vorzugsweise Dioxan, in Gegenwart katalytischer Mengen starker Mineralsäuren oder organischer Säuren, vorzugsweise p-Toluolsulfonsäure, jeweils in der Siedehitze durchgeführt.

Die Umsetzung der Amine mit geschützter Sulfamoylgruppe (B = Gruppierung III) führt in den meisten Fällen zu höheren Ausbeuten als bei Verwendung der Amine mit freier Sulfamoylgruppe und ist daher vorzuziehen.

Die so erhaltenen Verbindungen der allgemeinen Formel VI werden gegebenenfalls durch Zugabe von Wasser gefällt. Bevorzugt wird nun mit Alkalimetallhydroxiden hydrolysiert. Durch nachfolgendes Ansäuern bis pH 2–4 können die Verbindungen der Formel VI ($R^6$ = H) isoliert werden.

Die Reduktion der Verbindungen der allgemeinen Formel VI, gegebenenfalls auch vor der Hydrolyse, erfolgt vorteilhaft durch katalytische Hydrierung. Als Katalysatoren kommen die für die katalytische Hydrierung üblichen Katalysatoren in Frage, z.B. Pd/Aktivkohle, $PtO_2$, Rh/Aktivkohle oder Pt/Aktivkohle. Die Hydrierungen werden bei 1–150 atm. Wasserstoffdruck, vorzugsweise bei 20–100 atm, sowie bei Raumtemperatur bis 150 °C, vorteilhaft bei 60–100 °C, durchgeführt. Als Lösungsmittel dienen die bei katalytischen Hydrierungen üblicherweise benutzten Lösungsmittel, z.B. organische Säuren wie Eisessig, organische Alkohole, Äther oder Ester wie beispielsweise Methanol, Dioxan oder Essigester.

Die Isolierung der reduzierten Verbindungen richtet sich nach der Art des Lösungsmittels und geschieht in vielen Fällen dadurch, dass man sie mit einem Nichtlösungsmittel, wie etwa Wasser oder Kohlenwasserstoffen wie Petroläther, ausfällt oder alternativ das benutzte Lösungsmittel abdampft.

Die Reduktion kann auch mit anderen für Pyrrole bekannten Reduktionsmitteln, wie z.B. Eisessig/Zink, oder Jodwasserstoffsäure und rotem Phosphor erfolgen.

Bedeuten $R^4$ und $R^{4'}$ OH, SH und/oder $NH_2$-Gruppen, so können diese in Verbindungen der Formel VI geschützt sein, beispielsweise durch eine Acylgruppe oder einen Benzylrest. Bei der katalytischen Hydrierung bzw. der Hydrolyse von Verbindungen der allgemeinen Formel VI werden diese Schutzgruppen dann gleichzeitig mit abgespalten.

Ausser den in den Beispielen beschriebenen Verbindungen kann man die nachstehend genannten herstellen:

4-Phenoxy-3-(2,5-diethyl-1-pyrrolidinyl)-5-sulfamoyl-benzoesäure.

4-(4-Fluorphenoxy)-3-(2,5-dimethyl-1-pyrrolidinyl)-5-sulfamoyl-benzoesäure.

4-(4-Trifluormethylphenoxy)-3-(2,5-dimethyl-1-pyrrolidinyl)-5-sulfamoyl-benzoesäure.

4-(4-Hydroxyphenoxy)-3-(2,5-dimethyl-1-pyrrolidinyl)-5-sulfamoyl-benzoesäure.

4-(4-Aminophenoxy)-3-(2-methyl-1-pyrrolidinyl)-5-sulfamoyl-benzoesäure.

4-Phenylthio-3-(2,5-dimethyl-1-pyrrolidinyl)-5-sulfamoyl-benzoesäure.

4-Anilino-3-(2,5-dimethyl-1-pyrrolidinyl)-5-sulfamoyl-benzoesäure.

4-(4-Methylanilino)-3-(2,5-dimethyl-1-pyrrolidinyl)-5-sulfamoyl-benzoesäure.

4-Phenyl-3-(2,5-diethyl-1-pyrrolidinyl)-5-sulfamoyl-benzoesäure.

4-(4-Methoxyphenyl)-3-(2,5-dimethyl-1-pyrrolidinyl)-5-sulfamoyl-benzoesäure.

4-(4-Methylphenyl)-3-(2,4-dimethyl-1-pyrrolidinyl)-5-sulfamoyl-benzoesäure.

4-(3-Thienyl)-3-(2,5-dimethyl-1-pyrrolidinyl)-5-sulf-amoyl-benzoesäure.

4-(3,4-Methylendioxyphenoxy)-3-(2,5-dimethyl-1-pyrrolidinyl)-5-sulfamoyl-benzoesäure.

4-(3-Dimethylaminophenoxy)-3-(2,5-dimethyl-1-pyrrolidinyl)-5-sulfamoyl-benzoesäure.

4-(3-Methylphenoxy)-3-(2-ethyl-1-pyrrolidinyl)-5-sulfamoyl-benzoesäure.

4-(4-Methylphenoxy)-3-(2,4-dimethyl-1-pyrrolidinyl)-5-sulfamoyl-benzoesäure.

4-Phenoxy-3-(2,3,4-trimethyl-1-pyrrolidinyl)-5-sulfamoyl-benzoesäure.

4-(4-Methylphenoxy)-3-(2,3,5-trimethyl-1-pyrrolidinyl)-5-sulfamoyl-benzoesäure.

4-Phenoxy-3-(2,3,4,5-tetramethyl-1-pyrrolidinyl)-5-sulfamoyl-benzoesäure.

4-(4-Methylphenoxy)-3-(2,3-dimethyl-1-pyrrolidinyl)-5-sulfamoyl-benzoesäure.

Im Vergleich zu den aus der DE-OS 2 419 970 bekannten salidiuretisch wirksamen in 3-Stellung pyrrolidinylsubstituierten 5-Sulfamoylbenzoesäurederivaten weisen die erfindungsgemässen Verbindungen eine verbesserte salidiuretische Wirkung auf. Dies ist insofern überraschend, da eine Einführung von Alkylgruppen in salidiuretisch wirksame Pyrrol-Verbindungen (DE-OS 2 756 783) eine Verminderung der salidiuretischen Aktivität bewirkt.

Die erfindungsgemässen Sulfamoylbenzoesäurederivate der Formel I sowie deren pharmazeutisch verträgliche Salze sind hochwirksame Diuretika und Saludiuretika, die in der Human- und Veterinärmedizin eingesetzt werden können. Auch die als Zwischenprodukte beschriebenen Pyrrole der allgemeinen Formel VI ($R^6$ = H und B = NHR) können hochwirksame Pharmazeutika, insbesondere Diuretika und Saluretika sein und therapeutisch verwendet werden.

Die Verbindungen I bzw. VI (B = NHR) werden in Dosierungen von 0,5 bis 100 mg in Kapseln, Dragees, Tabletten oder Lösungen mit verschiedenen Zusätzen enteral z.B. oral mit Sonde oder dergleichen oder parenteral (Injektion in das Gefässsystem, z.B. intravenös oder auch Injektion in die Muskulatur oder unter die Haut und dgl.) verabfolgt. Sie eignen sich zur Behandlung von Ödemkrankheiten, wie kardiale, renale oder hepatisch bedingter Ödeme und anderer auf Störungen des Wasser- und Elektrolyt-Haushalts zurückzuführender Erscheinungen. Die Verbindungen können allein oder in Kombination mit anderen salidiuretisch wirksamen Substanzen auch anderer Wirkungsrichtung angewendet werden, oder mit verschiedenen anderen Arzneimitteln getrennt, alternierend oder in Kombination verabfolgt werden. Insbesondere sind zu nennen Spironolacton, Triameteren, Amilorid und andere $K^+$-retinierende Verbindungen alternierend mit langwirkenden Salidiuretika vom Typ des Chlorthalidons oder anderen $K^+$-Verlust substituierenden Kalium-enthaltenden Verbindungen (Salze oder dgl.).

Beispiel 1

4-(4-Methylphenoxy)-3-(2,5-dimethyl-1-pyrrolidinyl)-5-sulfamoyl-benzoesäure

a) 3-N-(2,5-Dimethylpyrrolo)-4-(4-methylphenoxy)-5-N,N-dimethylaminomethylenaminosulfonyl-benzoesäuremethylester

25 g (0,064 Mol) 3-Amino-4-(4-methylphenoxy)-5-N,N-dimethylaminomethylenaminosulfonylbenzoesäuremethylester werden in 250 ml Eisessig und 20 ml (0,168 Mol) Acetonylaceton 1 Stunde unter Rückfluss erhitzt. Die Lösung wird in Eiswasser eingerührt und das ausgefallene Produkt aus Methanol umkristallisiert.

Weisse Kristalle vom Schmp. 190–192 °C

b) 3-N-(2,5-Dimethylpyrrolo)-4-(4-methylphenoxy)-5-sulfamoyl-benzoesäure

22 g (0,047 Mol) 3-N-(2,5-Dimethylpyrrolo)-4-(4-methylphenoxy)-5-N,N-dimethylaminomethylenaminosulfonylbenzoesäuremethylester werden in 250 ml 2n NaOH suspendiert und bis zur klaren Lösung unter Rückfluss erhitzt. Neutralisieren der Lösung mit 2n HCl lässt die freie Säure ausfallen. Umkristallisation aus Ether/Petrolether.

Schmp. 207–210 °C

c) 4-(4-Methylphenoxy)-3-(2,5-dimethyl-1-pyrrolidinyl)-5-sulfamoyl-benzoesäure

10 g (0,025 Mol) 3-N-(2,5-Dimethylpyrrolo)-4-(4-methylphenoxy)-5-sulfamoyl-benzoesäure werden in 200 ml Methanol gelöst, mit 7 g 10%iger Palladium/Kohle versetzt und bei 70 °C, 100 atm. 12 Stunden lang hydriert. Die Lösung wird filtriert, eingeengt und mit Wasser versetzt. Das Produkt kristallisiert aus.

Weisse Kristalle vom Schmp. 191–193 °C

Beispiel 2

4-(4-Methylphenoxy)-3-(2-methyl-1-pyrrolidinyl)-5-sulfamoyl-benzoesäure

a) 3-N-(2-Methylpyrrolo)-4-(4-methylphenoxy)-5-N,N′-dimethylaminomethylenaminosulfonylbenzoesäuremethylester

10 g (0,026 Mol) 3-Amino-4-(4-methylphenoxy)-5-N,N′-dimethylaminomethylenaminosulfonylbenzoesäuremethylester werden in einer Mischung aus 50 ml Methylenchlorid und 50 ml Eisessig gelöst und mit 7 ml (0,048 Mol) 2,5-Dimethyoxy-2-methyltetrahydrofuran versetzt. Die Mischung wird eine Stunde bei Raumtemperatur gerührt und anschliessend mit Wasser versetzt. Die $CH_2Cl_2$-Phase wird mehrfach mit Wasser gewaschen, mit $Na_2SO_4$ getrocknet und eingeengt. Nach Zusatz von Ether kristallisiert das Produkt aus.

Weisse Kristalle vom Schmp. 215 °C

b) 3-N-(2-Methylpyrrolo)-4-(4-methylphenoxy)-5-sulfamoyl-benzoesäure

11,1 g (0,024 Mol) 3-N-(2-Methylpyrrolo)-4-(4-methylphenoxy)-5-N,N-dimethylaminomethylenaminosulfonylbenzoesäuremethylester werden in 150 ml 2n NaOH suspendiert und bis zur klaren Lösung unter Rückfluss erhitzt. Ansäuern mit 2n HCl lässt die freie Säure ausfallen. Umkristallisation aus Methanol/Wasser.

Schmp. 217–219 °C

c) 4-(4-Methylphenoxy)-3-(2-methyl-1-pyrrolidinyl)-5-sulfamoy-benzoesäure

8,2 g (0,024 Mol) 3-N-(2-Methylpyrrolo)-4-(4-methylphenoxy)-5-sulfamoyl-benzoesäure werden in 150 ml Methanol gelöst, mit 4 g 10%iger Palladium/Kohle versetzt und 10 Stunden bei 70 °C und 100 atm. hydriert. Die Lösung wird filtriert, zur Trockne eingedampft und der Rückstand aus Methanol/Wasser umkristallisiert.

Weisse Kristalle vom Schmp. 234–236 °C

Beispiel 3

4-Phenoxy-3-(2-methyl-1-pyrrolidinyl)-5-sulfamoyl-benzoesäure

a) 3-N-(2-Methylpyrrolo)-4-phenoxy-5-N,N-dimethylaminomethylenaminosulfonyl-benzoesäuremethylester

12,2 g (0,032 Mol) 3-Amino-4-phenoxy-5-N,N-dimethylaminomethylenaminosulfonyl-benzoesäuremethylester werden in 150 ml Eisessig zum Rückfluss erhitzt und mit 7 ml (0,048 Mol) 2,5-Dimethoxy-2-methyl-tetrahydrofuran versetzt. Nach 15 Minuten Reaktionsdauer wird die Mischung in Eiswasser eingerührt und das ausgefallene Produkt aus Methanol umkristallisiert.

Schmp.: 188–189 °C

b) 3-N-(2-Methylpyrrolo)-4-phenoxy-5-sulfamoylbenzoesäure

10 g (0,023 Mol) 3-N-(2-Methylpyrrolo)-4-phenoxy-5-N,N-dimethylaminomethylenaminosulfonylbenzoesäuremethylester in 150 ml 2n NaOH suspendiert und bis zur klaren Lösung unter Rückfluss erhitzt. Durch Ansäuern mit 2n HCl erhält man die freie Säure. Umkristallisation aus Methanol.

Schmp.: 265–266 °C

c) 4-Phenoxy-3-(2-methyl-1-pyrrolidinyl)-5-sulfamoylbenzoesäure

13 g (0,035 Mol) 3-N-(2-Methylpyrrolo)-4-phenoxy-5-sulfamoylbenzoesäure werden in 200 ml Methanol gelöst, mit 1 g 10%iger Palladium/Kohle versetzt und 8 Stunden bei 100 °C und 100 atm. hydriert. Die Lösung wird filtriert und das Lösungsmittel abgezogen. Der Rückstand wird aus Methanol/Wasser umkristallisiert.

Weisse Kristalle vom Schmp.: 221–223 °C

Beispiel 4

4-Phenoxy-3-(2,5-dimethyl-1-pyrrolidinyl)-5-sulfamoyl-benzoesäure

a) 3-N-(2,5-Dimethylpyrrolo)-4-phenoxy-5-N,N-dimethylaminomethylenaminosulfonyl-benzoesäuremethylester

25 g (0,067 Mol) 3-Amino-4-phenoxy-5-N,N-dimethylaminomethylenaminosulfonyl-benzoesäuremethylester werden in 150 ml Eisessig zum Sieden erhitzt und mit 17 ml (0,116 Mol) Acetonylaceton versetzt. Nach 30 Minuten Reaktionsdauer wird die Mischung in Eiswasser eingerührt und das ausgefallene Produkt abgesaugt. Umkristallisation aus Methanol.

Schmp.: 198 °C

b) 3-N-(2,5-Dimethylpyrrolo)-4-phenoxy-5-sulfamoylbenzoesäure

10 g (0,022 Mol) 3-N-(2,5-Dimethylpyrrolo)-4-phenoxy-5-N,N-dimethylaminomethylenaminosulfonyl-benzoesäuremethylester werden analog zu Beispiel 3b verseift. Umkristallisation aus Methanol.

Schmp.: 280–292 °C (Zers.)

c) 4-Phenoxy-3-(2,5-dimethyl-1-pyrrolidinyl)-5-sulfamoyl-benzoesäure

14,5 g (0,038 Mol) 3-N-(2,5-Dimethylpyrrolo)-4-phenoxy-5-sulfamoylbenzoesäure werden in 200 ml Methanol gelöst, mit 1 g 10%iger Palladium/Kohle versetzt und 8 Stunden bei 100 °C und 100 atm. hydriert. Es wird filtriert, vom Lösungsmittel befreit und aus Methanol/Wasser umkristallisiert.

Schmp.: 207–210 °C (Zers.)

Beispiel 5

4-(4-Methylphenoxy)-3-(2-ethyl-5-methyl-1-pyrrolidinyl)-5-sulfamoylbenzoesäure

a) 3-N-(2-Ethyl-5-methylpyrrolo)-4-(4-methylphenoxy)-5-N,N-dimethylaminomethylenaminosulfonyl-benzoesäuremethylester

10 g (0,026 Mol) 3-Amino-4-(4-methylphenoxy)-5-N,N-dimethylaminomethylenaminosulfonyl-benzoesäuremethylester werden in 60 ml Dioxan gelöst, mit 50 mg p-Toluol-sulfonsäure sowie mit 10 ml (0,076 Mol) Heptan-2,5-dion versetzt. Die Mischung wird 2 Stunden unter Rückfluss erhitzt und in Eiswasser eingerührt. Das ausgefallene Produkt wird aus Methanol umkristallisiert.

Schmp.: 192–195 °C

b) 3-N-(2-Ethyl-5-methylpyrrolo)-4-(4-Methylphenoxy)-5-sulfamoyl-benzoesäure

9,1 g (0,019 Mol) 3-N-(2-Ethyl-5-methylpyrrolo)-4-(4-methylphenoxy)-5-N,N-dimethylaminomethylenaminosulfonyl-benzoesäuremethylester werden in 100 ml 2n NaOH suspendiert und bis zur klaren Lösung am Rückfluss erhitzt. Durch Ansäuern auf pH 1–2 fällt die freie Säure aus. Umkristallisation aus Methanol/$H_2O$.

Schmp.: 228–231 °C

c) 4-(4-Methylphenoxy)-3-(2-ethyl-5-methyl-1-pyrrolidinyl)-5-sulfamoylbenzoesäure

3,2 g (0,008 Mol) 3-N-(2-Ethyl-5-methylpyrrolo)-4-(4-methylphenoxy)-5-sulfamoylbenzoesäure werden in 100 ml Methanol gelöst und unter Zusatz von 1,7 g 10%iger Palladium/Kohle bei Raumtemperatur und 100 atm. hydriert. Man filtriert vom Katalysator, engt bis zur Trockne ein und versetzt mit Petrolether 40–60/etwas Toluol. Das Produkt kristallisiert aus.

Umkristallisation aus Methanol/$H_2O$.

Schmp.: 198–200 °C

Beispiel 6

4-(4-Methoxyphenoxy)-3-(2,5-dimethyl-1-pyrrolidinyl)-5-sulfamoylbenzoesäure

a) 3-N-(2,5-Dimethylpyrrolo)-4-(4-methoxyphenoxy)-5-N,N-dimethylaminomethylenaminosulfonyl-benzoesäuremethylester

20,0 g (0,049 Mol) 3-Amino-4-(4-methylphenoxy)-5-N,N-dimethylaminomethylenaminosulfonyl-benzoesäuremethylester und 12 ml (0,1 Mol) Acetonylaceton werden in 200 ml Eisessig am Rückfluss erhitzt. Nach 45 Minuten Reaktionszeit wird die Mischung in Eiswasser eingerührt. Das ausgefallene Produkt wird aus Methylenchlorid/Diethylether umkristallisiert.

Farblose Kristalle vom Schmp.: 214–216 °C

b) 3-N-(2,5-Dimethylpyrrolo)-4-(4-methoxyphenoxy)-5-sulfamoyl-benzoesäure

5 g (0,010 Mol) 3-N-(2,5-Dimethylpyrrolo)-4-(4-methoxyphenoxy)-5-N,N-dimethylaminomethylenaminosulfonyl-benzoesäuremethylester werden in 50 ml 2n NaOH suspendiert und bis zur klaren Lösung am Rückfluss erhitzt. Durch Ansäuern mit 2n HCl auf pH 5–6 fällt die freie Säure aus. Umkristallisation aus $CH_3OH/H_2O$.

Weisse Kristalle vom Schmp.: 207–209 °C

c) 4-(4-Methoxyphenoxy)-3-(2,5-dimethyl-1-pyrrolidinyl)-5-sulfamoylbenzoesäure

3,8 g (0,009 Mol) 3-N-(2,5-Dimethylpyrrolo)-4-(4-methoxyphenoxy)-5-sulfamoylbenzoesäure werden in 50 ml Methanol gelöst und 8 Stunden bei 100 °C und 100 atm. unter Zusatz von 500 mg 10%iger Palladium/Aktivkohle hydriert. Man filtriert den Katalysator ab und engt die Lösung am Rotationsverdampfer bis zur beginnenden Kristallisation ein.

Rosastichige Kristalle vom Schmp.: 179–181 °C

Beispiel 7

4-(4-Methylphenoxy)-3-(2-ethyl-1-pyrrolidinyl)-5-sulfamoyl-benzoesäure

a) 3-N-(2-Ethylpyrrolo)-4-(4-methylphenoxy)-5-N,N-dimethylaminomethylenaminosulfonyl-benzoesäuremethylester

10 g (0,026 Mol) 3-Amino-4-(4-methylphenoxy)-5-N,N-dimethylaminomethylenaminosulfonyl-benzoesäure werden in einer Mischung aus 75 ml Eisessig und 75 ml Methylenchlorid gelöst. Man erhitzt 30 Minuten lang unter Rückfluss und tropft während dieser Zeit 8 ml (0,05 Mol) 2,5-Dimethoxy-2-ethyl-tetrahydrofuran dazu. Nach weiteren 10 Minuten Rückflusskochen wird die Lösung mit Wasser gewaschen, eingeengt und mit Diethylether versetzt. Das Produkt kristallisiert aus.

Weisse Kristalle vom Schmp.: 193–195 °C

b) 3-N-(2-Ethylpyrrolo)-4-(4-methylphenoxy)-5-sulfamoylbenzoesäure

12,0 g (0,026 Mol) 3-N-(2-Ethylpyrrolo)-4-(4-methylphenoxy) -5-N,N'-dimethylaminomethylen-aminosulfonyl-benzoesäuremethylester werden in 150 ml 2n NaOH suspendiert und bis zur klaren Lösung erhitzt. Das Produkt wird durch Ansäuern mit 2n HCl auf pH 5–6 ausgefällt. Umkristallisation aus Methanol/$H_2O$.

Schmp.: 236–237 °C

c) 4-(4-Methylphenoxy)-3-(2-ethyl-1-pyrrolidinyl)-5-sulfamoylbenzoesäure

6,2 g (0,015 Mol) 3-N-(2-Ethylpyrrolo)-4-(4-methylphenoxy)-5-sulfamoylbenzoesäure werden in 100 ml Methanol gelöst und unter Zusatz von 3 g 10%iger Palladium/Kohle bei 70 °C und 100 atm. 14 Stunden lang hydriert. Die Lösung wird filtriert, zur Trockne eingeengt und der Rückstand aus Methanol/$H_2O$ umkristallisiert.

Schmp.: 235–237 °C

Beispiel 8

4-(4-Methylanilino)-3-(2,5-dimethyl-1-pyrrolidinyl)-5-sulfamoylbenzoesäure

a) 3-N-(2,5-Dimethylpyrrolo)-4-(4-methylanilino)-5-N,N-dimethylaminomethylenaminosulfonyl-benzoesäuremethylester

20 g (0,051 Mol) 3-Amino-4-(4-Methylanilino)-5-N,N-dimethylaminomethylenaminosulfonyl-benzoesäuremethylester werden in 150 ml Eisessig gelöst, mit 15 ml (0,126 Mol) Acetonylaceton versetzt und 20 Minuten unter Rückfluss erhitzt. Die Mischung wird in Eiswasser eingerührt, das ausgefallene Produkt aus Methanol/$H_2O$ umkristallisiert.

Farblose Kristalle vom Schmp. 178–180 °C

b) 3-N-(2,5-Dimethylpyrrolo)-4-(4-methylanilino)-5-sulfamoylbenzoesäure

12,0 g (0,026 Mol) 3-N-(2,5-Dimethylpyrrolo)-4-(4-methylanilino)-5-N,N-dimethylaminomethylen-aminosulfonyl-benzoesäuremethylester werden in 150 ml 2n NaOH bis zur klaren Lösung unter Rückfluss erhitzt. Ansäuern mit 2n HCl lässt die freie Säure ausfallen. Umkristallisation aus Methanol/$H_2O$.

Schmp.: 212–214 °C

c) 4-(4-Methylanilino)-3-(2,5-dimethyl-1-pyrrolidinyl)-5-sulfamoylbenzoesäure

5,4 g (0,014 Mol) 3-N-(2,5-Dimethylpyrrolo)-4-(4-methylanilino)-5-sulfamoylbenzoesäure werden in 100 ml Methanol gelöst, mit 3 g 10%iger Palladium/Kohle versetzt und 16 Stunden lang bei 60 °C und 150 atm. hydriert. Nach Filtrieren und Abziehen des Lösungsmittels wird der Rückstand aus Toluol umkristallisiert.

Schmp. 212–214 °C

Beispiel 9

4-(4-Methoxyphenoxy)-3-(2-methyl-1-pyrrolidinyl)-5-sulfamoylbenzoesäure

a) 3-N-(2-Methylpyrrolo)-4-(4-methoxyphenoxy)5-N,N-dimethylaminomethylenaminosulfonyl-benzoesäuremethylester

8 g (0,020 Mol) 3-Amino-4-(4-Methoxyphenoxy)-5-N,N-dimethylaminomethylenaminosulfonyl-benzoesäuremethylester werden in einem Gemisch aus 60 ml Methylenchlorid und 60 ml Eisessig gelöst, mit 6 ml (0,04 Mol) 2,5-Dimethoxy-2-methyltetrahydrofuran versetzt und die Mischung 60 Minuten bei Raumtemperatur gerührt. Durch mehrfaches Waschen mit Wasser wird vom Eisessig abgetrennt, das Methylenchlorid abgezogen und der Rückstand aus Ether/wenig Methylenchlorid umkristallisiert.

Schmp.: 191–192 °C

b) 3-N-(2-Methylpyrrolo)-4-(4-methoxyphenoxy)-5-sulfamoylbenzoesäure

7,5 g (0,016 Mol) 3-N-(2-Methylpyrrolo)-4-(4-methoxyphenoxy)-5-N,N-dimethylaminomethylen-aminosulfonyl-benzoesäuremethylester werden in 100 ml 2n NaOH suspendiert und bis zur klaren Lösung unter Rückfluss erhitzt. Ansäuern auf pH 3–4 lässt die freie Säure ausfallen. Umkristallisation aus Methanol/Wasser

Schmp.: 193–195 °C

c) 4-(4-Methoxyphenoxy)-3-(2-methyl-1-pyrrolidinyl)-5-sulfamoylbenzoesäure

3,2 g (0,009 Mol) 3-N-(2-Methylpyrrolo)-4-(4-methoxyphenoxy)-5-sulfamoylbenzoesäure werden in 50 ml Eisessig gelöst, mit 1 g 10%iger Palladium/Kohle versetzt und 10 Stunden bei 80 °C und 20 atm. hydriert. Nach Filtrieren wird in Wasser eingerührt. Das Produkt kristallisiert aus.

Schmp.: 197–199 °C

Beispiel 10

4-(2-Thienyl)-3-(2,5-dimethyl-1-pyrrolidinyl)-5-sulfamoylbenzoesäure

a) 3-N-(2,5-Dimethylpyrrolo)-4-(2-thienyl)-5-N,N-dimethylaminomethylenaminosulfonylbenzoesäuremethylester

18,4 g (0,05 Mol) 3-Amino-4-(2-thienyl)-5-N,N,-dimethylaminomethylenaminosulfonylbenzoesäuremethylester werden in 150 ml Eisessig gelöst, mit 6,3 g (0,055 Mol) Acetonylaceton versetzt und 60 Minuten unter Rückfluss erhitzt. Man giesst auf Eiswasser und saugt das Produkt ab. Umkristallisation aus Äthanol unter Zusatz von Tierkohle.

Schmp.: 205–207 °C

b) 3-N-(2,5-Dimethyl-1-pyrrolidinyl)-4-(2-thienyl)-5-N,N-dimethylaminomethylenaminosulfonyl-benzoesäuremethylester

10 g «Pyrrolester» (10 a) werden in 250 ml Eisessig gelöst, mit 7,5 g 10%iger Palladiumkohle versetzt und 15 Stunden bei 80 °C und 100 atm. hydriert. Die Lösung wird filtriert, auf 100 ml eingeengt, mit 500 ml Eiswasser versetzt und erneut

filtriert. Die Lösung wird mit Essigsäureäthylester mehrfach extrahiert, die vereinigten Essigesterauszüge über Magnesiumsulfat getrocknet und bis zur Trockne eingeengt. Umkristallisation aus Isopropanol.

Kristalle vom Schmp.: 180–182 °C

c) 4-(2-Thienyl)-3-(2,5-dimethylpyrrolidinyl-5-sulfamoyl-benzoesäure

2,25 g (0,005 Mol) «Pyrrolidinester» (10 b) werden in 2n NaOH suspendiert und bis zur klaren Lösung unter Rückfluss erhitzt. Ansäuern mit 2n HCl auf pH 1 lässt die freie Säure ausfallen.

Kristalle vom Schmp.: 220–222 °C

Beispiel 11

4-(3-Pyridyloxy)-3-(2,5-dimethyl-1-pyrrolidinyl)-5-sulfamoylbenzoesäure

a) 5-Dimethylaminomethylenaminosulfonyl-3-nitro-4-(3-pyridyloxy)-benzoesäuremethylester

Eine Lösung von 200 g (0,57 Mol) 4-Chlor-5-dimethylaminomethylenaminosulfonyl-3-nitrobenzoesäuremethylester und 93 g (0,7 Mol) Kalium-3-oxypyridin in 1 l DMF wird 1,5 Stunden bei 80 °C gerührt und nach dem Abkühlen unter kräftigem Rühren in 4–5 l Eiswasser eingetragen. Das ausgefallene Produkt wird abgesaugt, mit $H_2O$ gewaschen und aus DMF/$CH_3OH$ umkristallisiert.

Kristalle vom Schmp.: 171–173 °C

b) 3-Amino-5-dimethylaminomethylenaminosulfonyl-4-(3-pyridyloxy)-benzoesäuremethylester

150 g 5-Dimethylaminomethylenaminosulfonyl-3-nitro-4-(3-pyridyloxy)-benzoesäuremethylester werden mit Raneynickel als Katalysator in Dimethylformamid bei 50 °C und 50 atm. 15 Stunden im Autoklav hydriert. Danach wird filtriert, das Filtrat eingeengt und der Rückstand aus DMF/$CH_3OH$ umkristallisiert.

Kristalle vom Schmp.: 234 °C

c) 3-N-(2,5-Dimethylpyrrolo)-4-(3-pyridyloxy)-5-N,N-dimethylaminomethylenaminosulfonyl-benzoesäuremethylester

Zu 20 g (0,053 Mol) 3-Amino-5-dimethylaminomethylenaminosulfonyl-4-(3-pyridyloxy)-benzoesäuremethylester in 200 ml Eisessig wird in der Siedehitze 19 ml (0,16 Mol) Acetonylaceton gegeben. Nach zweistündigem Rückfluss (DC-Kontrolle) wird die erhaltene Lösung in Eiswasser eingetragen. Bei Zusatz von 5–10 ml 2n NaOH fällt ein violett gefärbter Niederschlag aus. Das Rohprodukt wird mit Methanol ausgekocht und aus Aceton umkristallisiert.

Cremefarbene Kristalle vom Schmp.: 219–220 °C

d) 3-N-(2,5-Dimethyl-pyrrolo)-4-(3-pyridyloxy)-5-sulfamoyl-benzoesäure

15,3 g des bei (11c) erhaltenen Esters werden in 1n NaOH suspendiert und bis zur klaren Lösung am Rückfluss erhitzt (30–40 Minuten). Danach fällt man die freie Säure in der Kälte aus.

Kristalle vom Schmp.: 293–294 °C

e) 4-(3-Pyridyloxy)-3-(2,5-dimethyl-1-pyrrolidinyl)-5-sulfamoylbenzoesäure

12 g 3-N-(2,5-Dimethyl-pyrrolo)-4-(3-pyridyloxy-5-sulfamoyl-benzoesäure in 240 ml Eisessig werden mit 3 g Pd/C (10%) bei 100 °C und 20 atm. Wasserstoff 12 Stunden im Autoklav hydriert. Danach wird filtriert, das Filtrat eingeengt und der Rückstand $CH_3OH/H_2O$ umkristallisiert.

Kristalle vom Schmp.: 247–248 °C

Beispiel 12

4-(3-Pyridyloxy)-3-(2-methyl-1-pyrrolidinyl)-5-sulfamoylbenzoesäure

a) 3-N-(2-Methylpyrrolo)-4-(3-pyridyloxy)-5-N,N-dimethylaminomethylenaminosulfonylbenzoesäuremethylester

Zu 20 g (0,052 Mol) 3-Amino-5-dimethylaminomethylenaminosulfonyl-4-(3-pyridyloxy)-benzoesäuremethylester in einer Lösung von 100 ml Eisessig und 100 ml Methylenchlorid werden in der Siedehitze 15 ml (0,102 Mol) 2,5-Dimethoxy-2-methyltetrahydrofuran gegeben. Nach 10minutigem Kochen unter Rückfluss wird abgekühlt (DC-Kontrolle) und in 500 ml Eiswasser eingetragen. Die Methylenchlorid-Schich wird abgetrennt, die wässrige Lösung noch einmal mit 100 ml Methylenchlorid extrahiert und die vereinigten getrockneten Methylenchloridextrakte eingedampft und der Rückstand mit Äther angerieben.

Kristalle vom Schmp.: 194–196 °C

b) 3-N-(2-Methylpyrrolo)-4-(3-pyridyloxy)-5-sulfamoyl-benzoesäure

11,6 g des bei (12a) erhaltenen Esters werden in 1n NaOH suspendiert und bis zur klaren Lösung am Rückfluss erhitzt. Die freie Säure wird nach Abkühlen und Filtrieren der Lösung mit 4n HCl (pH 2–3) gefällt.

Kristalle vom Schmp.: 284–285 °C

c) 4-(3-Pyridyloxy)-3-(2-methyl-1-pyrrolidinyl)-5-sulfamoylbenzoesäure

Analog Beispiel 11e.

Kristalle vom Schmp.: 264–265 °C

Beispiel 13

4-(6-Methyl-3-pyridyloxy)-3-(2,5-dimethyl-1-pyrrolidinyl)-5-sulfamoylbenzoesäure

a) 5-Dimethylaminomethylenaminosulfonyl-4-(6-methyl-3-pyridyloxy)-3-nitro-benzoesäuremethylester

Analog Beispiel 11a, mit dem Kaliumsalz des 3-Hydroxy-6-methylpyridins

Hellgelbe Kristalle aus DMF/$CH_3OH$ vom Schmp.: 149– 150 °C

b) 3-Amino-5-dimethylaminomethylenaminosulfonyl-4-(6-methyl-3-pyridyloxy)-benzoesäuremethylester

Analog Beispiel 11b Umkristallisation aus Methanol

Schmp.: 150°

c) 3-N-(2,5-Dimethylpyrrolo)-4-(6-methyl-3-pyridyloxy)-5-N,N-dimethylaminomethylenaminosulfonyl-benzoesäuremethylester

Zu 29,6 g (0,075 Mol) «Aminester» (13b) in 300 ml Eisessig wird in der Siedehitze 26 ml (0,23 Mol) Acetonylaceton zugetropft. Nach 1½stündigem Rühren unter Rückfluss wird in 1 l Eiswasser eingetragen. Nach Zusatz von 2n NaOH fällt nach Stehen über Nacht im Eisschrank ein Niederschlag aus.

Kristalle vom Schmp.: 212–213 °C

d) 3-N-(2,5-Dimethylpyrrolo)-4-(6-methyl-3-pyridyloxy)-5-sulfamoyl-benzoesäure

20 g des bei 13c erhaltenen Esters werden in 150 ml 2n NaOH bis zur klaren Lösung am Rückfluss erhitzt und fällt die frei Säure in der Kälte mit 4n HCl aus.

Kristalle vom Schmp.: 292–293 °C (aus $CH_3OH$/$H_2O$)

e) 4-(6-Methyl-3-pyridyloxy)-3-(2,5-dimethyl-1-pyrrolidinyl)-5-sulfamoylbenzoesäure

10 g der unter 13d hergestellten Verbindung werden in 150 ml Eisessig in Gegenwart von 3 g Pd C (10%) bei 100° und 20 atm. Wasserstoff 15 Stunden im Autoklaven hydriert. Nach der Filtration wird eingeengt und der Rückstand aus $CH_3OH$/$H_2O$ umkristallisiert.

Kristalle vom Schmp.: 246–247 °C

Beispiel 14

4-(6-Methyl-3-pyridyloxy)-3-(2-methyl-1-pyrrolidinyl)-5-sulfamoylbenzoesäure

a) 3-N-(2-Methylpyrrolo)-4-(6-methyl-3-pyridyloxy)-5-sulfamoylbenzoesäure

Zu 19,6 g (0,05 Mol) 3-Amino-5-dimethylaminomethylenaminosulfonyl-4-(6-methyl-3-pyridyloxy)-benzoesäuremethylester in einer Lösung von 100 ml Eisessig und 100 ml Methylenchlorid wird in der Siedehitze 15 ml (0,1 Mol) 2,5-Dimethoxy-2-methyltetrahydrofuran gegeben. Nach 10minutigem Kochen unter Rückfluss wird abgekühlt und in 500 ml Eiswasser eingetragen. Die Methylenchloridschicht wird abgetrennt, die wässrige Lösung noch einmal mit 100 ml Methylenchlorid extrahiert und die vereinigten getrockneten Methylenchloridextrakte eingedampft. Der Rückstand wird mit Äther angerieben, abgesaugt, in 2n NaOH suspendiert und bis zur klaren Lösung am Rückfluss erhitzt. Die freie Säure wird nach Abkühlen und Filtrieren der Lösung mit 4n HCl gefällt.

Kristalle vom Schmp.: 176–178 °C (aus $CH_3OH$/$H_2O$)

b) 4-(6-Methyl-3-pyridyloxy)-3-(2-methyl-1-pyrrolidinyl)-5-sulfamoylbenzoesäure

18 g 3-N-(2-Methylpyrrolo)-4-(6-methyl-3-pyridyloxy)-5-sulfamoyl-benzoesäure werden in 350 ml Eisessig mit 6 g Pd/C (10%) bei 100° und 20 atm. Wasserstoff 15 Stunden im Autoklav hydriert. Nach der Filtration wird eingeengt und der Rückstand aus $CH_3OH$/$H_2O$ umkristallisiert.

Kristalle vom Schmp.: 253–254 °C

Beispiel 15

4-(4-Methylphenyl)-3N-(2,5-dimethyl-pyrrolo)-5-sulfamoyl-benzoesäure

a) 4-(4-Methylphenyl)-3-nitro-5-dimethylaminomethylenaminosulfonyl-benzoesäuremethylester

Eine Mischung aus 50 g (0,14 Mol) 4-Chlor-3-nitro-5-dimethylaminomethylenaminosulfonyl-benzoesäuremethylester, 70 g (0,51 Mol) 4-Bromtoluol und 42,8 g (0,68 Mol) Cu-Pulver werden 6 Stunden unter Stickstoff bei 170–175 °C gerührt; anschliessend auf 100 °C abgekühlt, mit 200 ml DMF verdünnt, von unlöslichen Kupfersalzen filtriert und mit 200 ml DMF nachgewaschen. Aus den vereinigten eingeengten DMF-Lösungen wird das Rohprodukt durch Zugabe von 250 ml Methanol gefällt.

Kristalle vom Schmp.: 170–173 °C (aus DMF/$CH_3OH$)

b) 3-Amino-4-(4-methylphenyl)-5-dimethylaminomethylenaminosulfonyl - benzoesäuremethylester

Eine Lösung von 28 g (0,07 Mol) 4-(4-Methylphenyl)-3-nitro-5-dimethylaminomethylenaminosulfonyl-benzoesäuremethylester in 200 ml DMF wird mit 3 g Raneynickel bei 50° und 50 atm. Wasserstoffdruck 10 Stunden im Autoklav hydriert. Nach Filtration wird in Eiswasser eingetragen, der Niederschlag abgesaugt und aus DMF/MeOH umkristallisiert.

Kristalle vom Schmp.: 225 °C

c) 4-(4-Methylphenyl-3N-(2,5-dimethyl-pyrrolo)-5-dimethylaminomethylenaminosulfonyl-benzoesäuremethylester

Zu einer Lösung von 15 g (0,04 Mol) 3-Amino-4-(4-methylphenyl)-5-dimethylaminomethylenaminosulfonyl-benzoesäuremethylester in 100 ml Eisessig wird in der Siedehitze 5 g Acetonylaceton zugetropft. Nach einstündigem Rühren unter Rückfluss (DC-Kontrolle) wird die erhaltene Lösung in Eiswasser eingetragen. Der Niederschlag wird abgesaugt und mit Wasser gewaschen.

Kristalle vom Schmp.: 225–228 °C

d) 4-(4-Methylphenyl)-3N-(2,5-dimethylpyrrolo)-5-sulfamoyl-benzoesäure

6 g bei 15c erhaltenen Esters werden in 1n NaOH suspendiert und bis zur klaren Lösung unter Rückfluss erhitzt. Unter Eiskühlung wird die freie Säure mit 2n HCl gefällt.

Kristalle vom Schmp.: 280–283 °C

**Patentansprüche für die Vertragsstaaten: BE, CH, DE, FR, GB, IT, LI, NL, SE**

1. Substituierte Pyrrolidinylsufamoylbenzoesäurederivate der allgemeinen Formel I

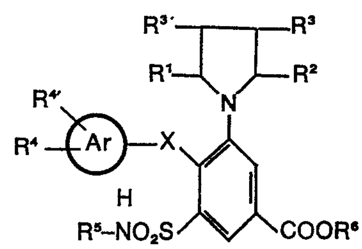

I

worin $R^1$ und $R^2$ gleich oder verschieden sind und Alkyl mit 1–4 C-Atomen bedeuten, wobei einer der Reste $R^1$ oder $R^2$ auch Wasserstoff sein kann,

$R^3$ und $R^{3'}$ gleich oder verschieden sind und Wasserstoff oder Alkyl mit 1–4 C-Atomen, oder zusammen eine C-C-Bindung bedeuten,

$R^4$ und $R^{4'}$ gleich oder verschieden sind und Wasserstoff, Halogen, $CF_3$, Alkyl oder Alkoxy mit 1–2 C-Atomen, Hydroxy, Amino oder Dimethylamino, in den verschiedenen Positionen des Arylkerns, oder zusammen die 3,4-Methylendioxygruppe bedeuten,

$R^5$ und $R^6$ gleich oder verschieden sind und Wasserstoff, Alkyl mit 1–4 C-Atomen oder Benzyl bedeuten, Ar Phenyl oder einen 5- oder 6gliedrigen hetero-aromatischen Ring mit einem O-, S- oder N-Atom bedeutet und X entfällt oder Sauerstoff, Schwefel, NH oder $CH_2$ bedeutet, sowie deren pharmazeutisch verträgliche Salze mit Basen oder Säuren.

2. Verfahren zur Herstellung der Verbindungen der Formel I, gemäss Anspruch 1, dadurch gekennzeichnet, dass man 3-Aminobenzoesäurederivate der allgemeinen Formel II

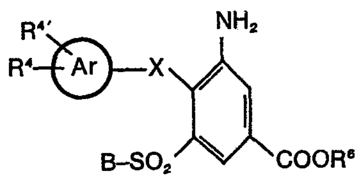

II

in der $R^4$, $R^{4'}$, $R^5$, $R^6$, X und Ar die angegebenen Bedeutungen haben, und B entweder die Gruppe $NHR^5$ oder die Gruppierung III

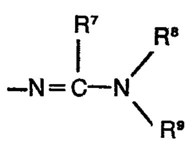

III

bedeutet, wobei die Reste $R^7$, $R^8$, $R^9$ für eine niedere Alkylgruppe stehen, $R^7$ aber auch Wasserstoff sein kann und/oder jeweils zwei der Substituenten auch cyclisch miteinander verbunden sein können, mit 2,5-Dialkoxytetrahydrofuranen der Formel IV

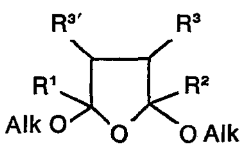

IV

oder mit 1,4-Diketonen der Formel V

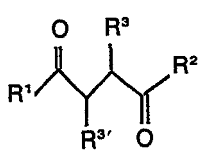

V

in der $R^1$, $R^2$, $R^3$ und $R^{3'}$ die angegebenen Bedeutungen haben und Alk für niederes Alkyl mit 1–4 C-Atomen steht, umsetzt und die erhaltenen Verbindungen der Formel VI

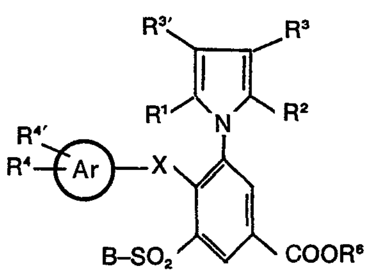

VI

in der die Reste $R^1$–$R^6$ und B die oben genannte Bedeutung haben, reduziert und vor oder nach der Reduktion gegebenenfalls hydrolysiert und erhaltene Verbindungen der Formel VI mit $R^6$ = H gegebenenfalls verestert und/oder die erhaltenen Verbindungen I in pharmazeutisch verträgliche Salze mit Säuren oder Basen überführt.

3. Pharmazeutische Zubereitungen mit salidiuretischer Wirkung, gekennzeichnet durch einen Gehalt an einer Verbindung der allgemeinen Formel I, gemäss Anspruch 1, gegebenenfalls mit üblichen pharmazeutischen Trägerstoffen und/oder Stabilisatoren.

4. Verbindungen der allgemeinen Formel I, gemäss Anspruch 1, zur Verwendung bei der Behandlung von Ödem-Krankheiten.

**Patentansprüche für den Vertragsstaat: AT**

1. Verfahren zur Herstellung von substituierten Pyrrolidinylsulfamoylbenzoesäurederivaten der allgemeinen Formel I

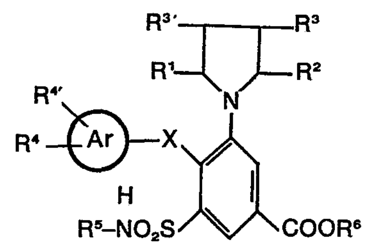

I

worin $R^1$ und $R^2$ gleich oder verschieden sind und Alkyl mit 1–4 C-Atomen bedeuten, wobei einer der Reste $R^1$ oder $R^2$ auch Wasserstoff sein kann,

$R^3$ und $R^{3'}$ gleich oder verschieden sind und Wasserstoff oder Alkyl mit 1–4 C-Atomen, oder zusammen eine C-C-Bindung bedeuten,

$R^4$ und $R^{4'}$ gleich oder verschieden sind und Wasserstoff, Halogen, $CF_3$, Alkyl oder Alkoxy mit 1–2 C-Atomen, Hydroxy, Amino oder Dimethylamino, in den verschiedenen Positionen des Arylkerns, oder zusammen die 3,4-Methylendioxygruppe bedeuten,

$R^5$ und $R^6$ gleich oder verschieden sind und Wasserstoff, Alkyl mit 1–4 C-Atomen oder Benzyl bedeuten, Ar Phenyl oder einen 5- oder 6gliedrigen hetero-aromatischen Ring mit einem O-, S- oder N-Atom bedeutet und X entfällt oder Sauerstoff, Schwefel, NH oder $CH_2$ bedeutet, sowie deren pharmazeutisch verträglichen Salze mit Basen oder Säuren, dadurch gekennzeichnet, dass man 3-Aminobenzoesäurederivate der allgemeinen Formel II

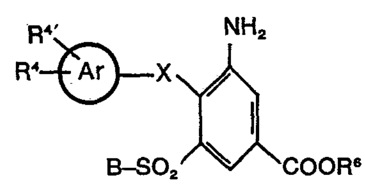

II

in der $R^4$, $R^{4'}$, $R^5$, $R^6$, X und Ar die angegebenen Bedeutungen haben, und B entweder die Gruppe $NHR^5$ oder die Gruppierung III

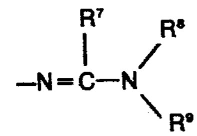

III

bedeutet, wobei die Reste $R^7$, $R^8$, $R^9$ für eine niedere Alkylgruppe stehen, $R^7$ aber auch Wasserstoff sein kann und/oder jeweils zwei der Substituenten auch cyclisch miteinander verbunden sein können, mit 2,5-Dialkoxytetrahydrofuranen der Formel IV

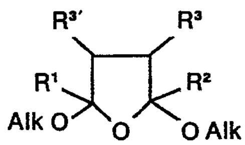

IV

oder mit 1,4-Diketonen der Formel V

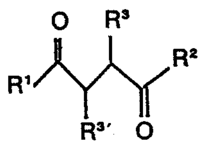

in der $R^1$, $R^2$, $R^3$ und $R^{3'}$ die angegebenen Bedeutungen haben und Alk für niederes Alkyl mit 1–4 C-Atomen steht, umsetzt und die erhaltenen Verbindungen der Formel VI

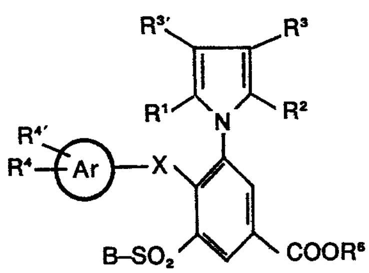

VI

in der die Reste $R^1$–$R^6$ und B die oben genannte Bedeutung haben, reduziert und vor oder nach der Reduktion gegebenenfalls hydrolysiert und erhaltene Verbindungen der Formel VI mit $R^6$ = H gegebenenfalls verestert und/oder die erhaltenen Verbindungen I in pharmazeutisch verträgliche Salze mit Säuren und Basen überführt.

2. Verfahren zur Herstellung pharmazeutischer Zubereitungen mit salidiuretischer Wirkung, dadurch gekennzeichnet, dass man eine Verbindung der allgemeinen Formel I, gemäss Anspruch 1, gegebenenfalls mit üblichen pharmazeutischen Trägerstoffen und/oder Stabilisatoren in eine pharmazeutisch geeignete Verabreichungsform bringt.

**Claims for the contracting States: BE, CH, LI, DE, FR, GB, IT, NL, SE**

1. Substituted pyrrolidinylsulfamoylbenzoic acid derivatives of the general formula I

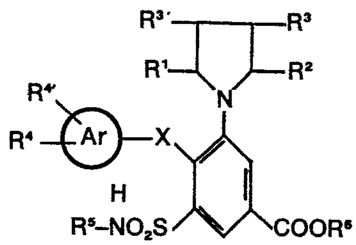

I

in which $R^1$ and $R^2$ are identical or different and denote alkyl with 1–4 C atoms and one of the radicals $R^1$ or $R^2$ can also be hydrogen, $R^3$ and $R^{3'}$ are identical or different and denote hydrogen or alkyl with 1–4 C atoms, or together denote a C-C bond, $R^4$ and $R^{4'}$ are identical or different and denote hydrogen, halogen, $CF_3$, alkyl or alkoxy with 1–2 C atoms, hydroxyl, amino or dimethylamino, in the various positions of the aryl nucleus, or together denote the 3,4-methylenedioxy group, $R^5$ and $R^6$ are identical or different and denote hydrogen, alkyl with 1–4 C atoms or benzyl, Ar denotes phenyl or a 5-membered or 6-membered hetero-aromatic ring containing a O, S or N atom and X is omitted or denotes oxygen, sulfur, NH or $CH_2$, and their pharmaceutically acceptable salts with bases or acids.

2. Process for the manufacture of the compounds of the formula I according to claim 1, which comprises reacting 3-amino-benzoic acid derivatives of the general formula II

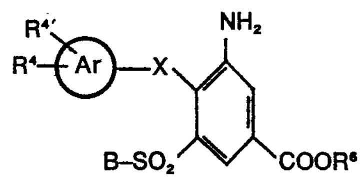

II

in which $R^4$, $R^{4'}$, $R^5$, $R^6$, X and Ar have the indicated meanings and B denotes either the group $NHR^5$ or the grouping III

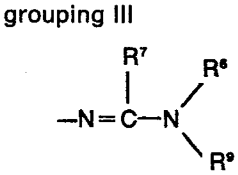

III

in which the radicals $R^7$, $R^8$ and $R^9$ represent a lower alkyl group but $R^7$ can also be hydrogen and/or any two of the substituents can also be cyclically bonded to one another, with 2,5-dialkoxytetrahydrofuranes of the formula IV

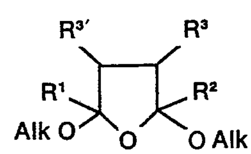

IV

or with 1,4-diketones of the formula V

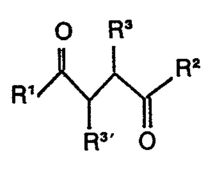

V

in which $R^1$, $R^2$, $R^3$ and $R^{3'}$ have the indicated meanings and Alk represents lower alkyl with 1–4 C atoms, and reducing the resulting compounds of the formula VI

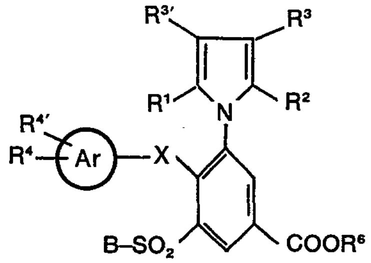

VI

in which the radicals $R^1$–$R^6$ and B have the meaning given above, and optionally hydrolyzing the said compounds, before or after the reduction, and optionally esterifying resulting compounds of the formula VI in which $R^6$ = H and/or converting the resulting compounds I into pharmaceutically acceptable salts with acids or bases.

3. Pharmaceutical formulations with a salidiuretic action, which contain a compound of the general formula I according to claim 1, optionally with conventional pharmaceutical excipients and/or stabilizers.

4. Compounds of the general formula I according to claim 1 for use in the treatment of oedomatous diseases.

**Revendications pour les Etats contractants: BE, CH, LI, DE, FR, GB, IT, NL, SE**

1. Dérivés substitués de l'acide pyrrolidinylsulfamoyl benzoïque répondant à la formule générale I

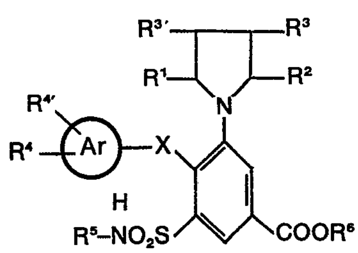

I

dans laquelle $R^1$ et $R^2$ sont identiques ou différents et représentent un alcoyle en $C_1$ à $C_4$, un des radicaux $R^1$ ou $R^2$ pouvant aussi être l'hydrogène,

$R^3$ et $R^{3'}$ sont identiques ou différents et désignent l'hydrogène ou un alcoyle en $C_1$ à $C_4$, ou ensemble une liaison C-C,

$R^4$ et $R^{4'}$ sont identiques ou différents et désignent l'hydrogène, un halogène, $CF_3$, un alcoyle ou un alcoxy en $C_1$ à $C_2$, un hydroxy, un amino ou un diméthylamino, dans les diverses positions du noyau aryle, ou ensemble le groupe 3,4-méthylènedioxy,

$R^5$ et $R^6$ sont identiques ou différents et désignent l'hydrogène, un alcoyle en $C_1$ à $C_4$ ou un benzyle, Ar un phényle ou un cycle hétéroaromatique à 5 ou 6 chaînons avec un atome de O, S ou N et X n'existe pas ou désigne l'oxygène, le soufre, NH ou $CH_2$, ainsi que leurs sels pharmaceutiquement acceptables avec des bases ou des acides.

2. Procédé de préparation des composés répondant à la formule I suivant la revendication 1, caractérisé en ce qu'on fait réagir des dérivés de l'acide 3-aminobenzoïque répondant à la formule générale II

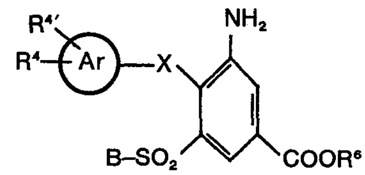

II

dans laquelle $R^4$, $R^{4'}$, $R^5$, $R^6$, X et Ar ont les significations indiquées et B désigne soit le groupe NHR $^5$, soit le groupe III

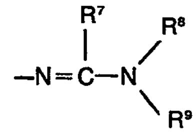

III

les radicaux $R^7$, $R^8$, $R^9$ désignent un groupe alcoyle inférieur, mais $R^7$ peut également être l'hydrogène et/ou à chaque fois deux des substituants peuvent aussi être liés entre eux cycliquement, avec des 2,5-dialcoxytétrahydrofuranes répondant à la formula IV

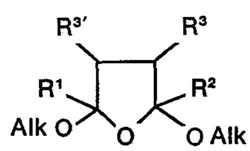

IV

ou avec des dicétones-1,4 répondant à la formule V

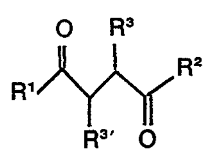

V

dans lesquelles $R^1$, $R^2$, $R^3$ et $R^{3p}$ ont les significations indiquées et Alk désigne un alcoyle en $C_1$ à $C_4$ et on réduit et hydrolyse éventuellement avant ou après la réduction, les composés obtenus répondant à la formule VI

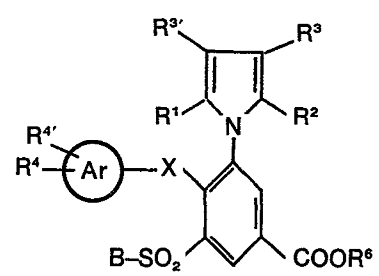

VI

dans laquelle les radicaux $R^1$–$R^6$ et B ont la signification indiquée ci-dessus, et on estérifie éventuellement les composés obtenus répondant à la formule VI dans laquelle $R^6$ = H et/ou on transforme les composés I obtenus en sels avec des acides ou des bases pharmaceutiquement acceptables.

3. Préparations pharmaceutiques douées d'une actions salidiurétique, caractérisées en ce qu'elles contiennent un composé répondant à la formule générale I suivant la revendication 1, le cas échéant avec des excipients et/ou des stabilisants pharmaceutiquement usuels.

4. Composés répondant à la formule générale I suivant la revendication 1 pour l'utilisation dans le traitement des maladies oedémateuses.

**Claims for the contracting State: AT**

1. Process for the manufacture of substituted pyrrolidinylsulfamoylbenzoic acid derivatives of the general formula I

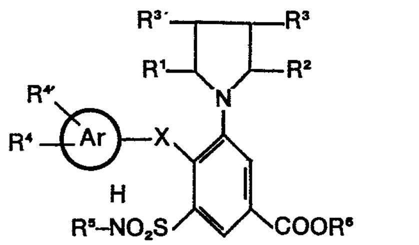

I

in which $R^1$ and $R^2$ are identical or different and denote alkyl with 1–4 C atoms and one of the radicals $R^1$ or $R^2$ can also be hydrogen, $R^3$ and $R^{3'}$ are identical or different and denote hydrogen or alkyl with 1–4 C atoms, or together denote a C-C bond, $R^4$ and $R^{4'}$ are identical or different and denote hydrogen, halogen, $CF_3$, alkyl or alkoxy with 1–2 C atoms, hydroxyl, amino or dimethylamino, in the various positions of the aryl nucleus, or together denote the 3,4-methylenedioxy group, $R^5$ and $R^6$ are identical or different and denote hydrogen, alkyl with 1–4 C atoms or benzyl, Ar denotes phenyl or a 5-membered or 6-membered hetero-aromatic ring containing a O, S or N atom and X is omitted or denotes oxygen, sulfur, NH or $CH_2$, and their pharmaceutically acceptable salts with bases or acids, characterized in reacting 3-aminobenzoic acid derivatives of the general formula II

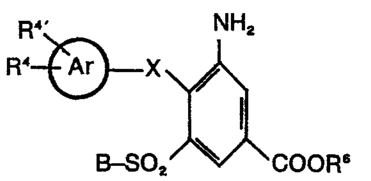

II

in which $R^4$, $R^{4'}$, $R^5$, $R^6$, X and Ar have the indicated meanings and B denotes either the group $NHR^5$ or the grouping III

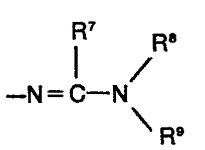

III

in which the radicals $R^7$, $R^8$ and $R^9$ represent a lower alkyl group but $R^7$ can also be hydrogen and/or any two of the substituents can also be cyclically bonded to one another, with 2,5-dialkoxytetrahydrofuranes of the formula IV

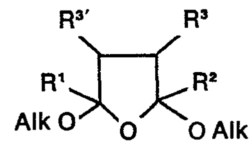

IV

or with 1,4-diketones of the formula V

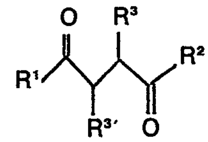

in which $R^1$, $R^2$, $R^3$ and $R^{3'}$ have the indicated meanings and Alk represents lower alkyl with 1–4 C atoms, and reducing the resulting compounds of the formula VI

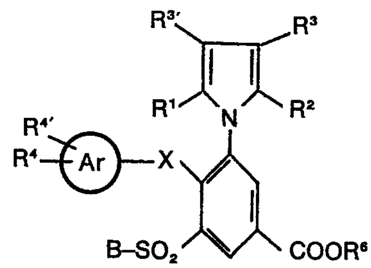

VI

in which the radicals $R^1$–$R^6$ and B have the meaning given above, and optionally hydrolyzing the said compounds, before or after the reduction, and optionally esterifying resulting compounds of the formula VI in which $R^6$ = H and/or converting the resulting compounds I into pharmaceutically acceptable salts with acids or bases.

2. Process for the manufacture of pharmaceutical formulations with a salidiuretic action, characterized in that a compound of the general formula I according to claim 1, optionally with conventional pharmaceutical excipients and/or stabilizers is brought into a pharmaceutically acceptable administering form.

**Revendications pour l'Etat contractant: AT**

1. Procédé de préparation de dérivés substitués de l'acide pyrrolidinylsulfamoyl benzoïque répondant à la formule générale I

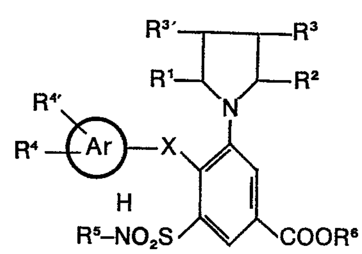

I

dans laquelle $R^1$ et $R^2$ sont identiques ou différents et représentent un alcoyle en $C_1$ à $C_4$, un des radicaux $R^1$ ou $R^2$ pouvant aussi être l'hydrogène,

$R^3$ et $R^{3'}$ sont identiques ou différents et désignent l'hydrogène ou un alcoyle en $C_1$ à $C_4$, ou ensemble une liaison C-C,

$R^4$ et $R^{4'}$ sont identiques ou différents et désignent l'hydrogène, un halogène, $CF_3$, un alcoyle ou un alcoxy en $C_1$ à $C_2$, un hydroxy, un amino ou un diméthylamino, dans les diverses positions du noyau aryle, ou ensemble le groupe 3,4-méthylènedioxy,

$R^5$ et $R^6$ sont identiques ou différents et désignent l'hydrogène, un alcoyle en $C_1$ à $C_4$ ou un benzyle, Ar un phényle ou un cycle hétéroaromatique à 5 ou 6 chaînons avec un atome de O, S ou N et X n'existe pas ou désigne l'oxygène, le soufre, NH ou $CH_2$, ainsi que leurs sels pharmaceutiquement acceptables avec des bases ou des acides, caractérisé en ce 9u'on fait réagir des dérivés de l'acide 3-aminobenzoïque répondant à la formule générale II

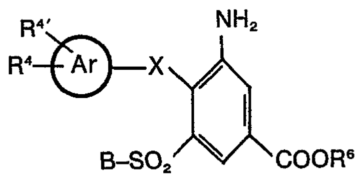

II

dans laquelle $R^4$, $R^{4'}$, $R^5$, $R^6$, X et Ar ont les significations indiquées et B désigne soit le groupe $NHR^5$, soit le groupe III

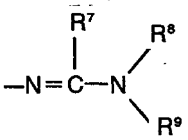

III

les radicaux $R^7$, $R^8$, $R^9$ désignent un groupe alcoyle inférieur, mais $R^7$ peut également être l'hydrogène et/ou à chaque fois deux des substituants peuvent aussi être liés entre eux cycliquement, avec des 2,5-dialcoxytétrahydrofuranes répondant à la formule IV

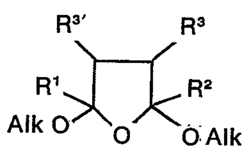

IV

ou avec des dicétones-1,4 répondant à la formule V

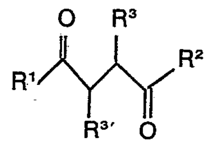

V

dans lesquelles $R^1$, $R^2$, $R^3$ et $R^{3'}$ ont les significations indiquées et Alk désigne un alcoyle en $C_1$ à $C_4$ et on réduit et hydrolyse éventuellement avant ou après la réduction, les composés obtenus répondant à la formule VI

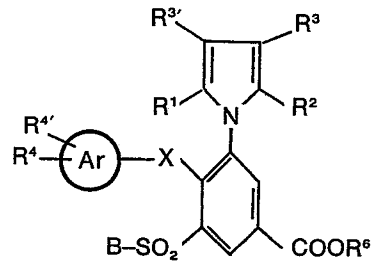

VI

dans laquelle des radicaux $R^1$–$R^6$ et B ont la signification indiquée ci-dessus, et on estérifie éventuellement les composés obtenus répondant à la formule VI dans laquelle $R^6$ = H et/ou on transforme les composés I obtenus en sels avec des acides ou des bases pharmaceutiquement acceptables.

2. Procédé de fabrication de préparations pharmaceutiques doués d'une action salidiurétique, caractérisé en ce qu'on met un composé répondant à la formule générale I suivant la revendication 1, le cas échéant avec des excipients et/ou des stabilisants pharmaceutiquement usuels sous une administration pharmaceutiquement appropriée.